# EUROPEAN PATENT APPLICATION

(11) **EP 4 721 690 A1**
(43) Date of publication of application: **08.04.2026**
(21) Application number: 24204886.6
(22) Date of filing: 07.10.2024
(51) Int. Cl.: A61B 34/20, A61M 25/09

(54) **ELONGATED DEVICE FOR MEDICAL INTERVENTIONAL APPLICATION**

(71) Applicant: Koninklijke Philips N.V., 5656 AG Eindhoven (NL)
(72) Inventor: MANK, Adrianus Johannes Gerardus, Eindhoven (NL); CLABBERS, Tom Johannes Hubertus, Eindhoven (NL); VAN DEN ENDE, Daan Anton, 5656AG Eindhoven (NL); VERSCHOOR, Volker Barnhart, Eindhoven (NL)
(74) Representative: Philips Intellectual Property & Standards

(57) **Abstract**

The present invention relates to an elongated device for medical interventional application, comprising a shell (12) comprising a longitudinal lumen (14). The shell (12) has a main section (20) and a distal terminal section (18). An optical fiber (16) is arranged in the lumen (14) and has a distal tip (17), the optical fiber (16) being configured for optical shape sensing. At least one of the shell (12) and the optical fiber (16) is longitudinally displaceable with respect to the other one of the shell (12) and the optical fiber (16). The device (10) comprises an actuating mechanism (50, 70) configured to displace the at least one of the shell (12) and the optical fiber (16) with respect to the other one of the shell (12) and the optical fiber (16) between an extended state, in which the distal tip (17) of the optical fiber (16) is within the distal terminal section (18) of the shell (12), and a retracted state, in which the distal tip (17) of the optical fiber (16) is in the main section (20) and out of the distal terminal section (18) of the shell (12). A system comprising the elongated device and a computer program are disclosed as well.

## Description

### FIELD OF THE INVENTION

The present invention generally relates to the field of optical shape sensing technology, also referred to as Fiber Optic RealShape (FORS) technology. In particular, the present invention relates to an elongated device for medical interventional application which is FORS enabled.

### BACKGROUND OF THE INVENTION

Fiber Optic RealShape (FORS) technology is a shape sensing and reconstruction technology used in elongated devices, like guidewires, to enable accurate, three-dimensional and real-time visualization of the devices to allow efficient navigation during minimally invasive endovascular procedures without the need for X-ray guidance. The reduction of X-ray radiation exposure during such a procedure is beneficial for both patients as well as physicians and staff.

The FORS technology uses a shape sensing enabled elongated device, like a guidewire, for use in the procedure and a measurement system, which typically comprises a light source and an optical detector compatible with the shape sensing enabled elongated device, as well as a signal processing unit and a user interface for viewing and tracking the visualized elongated device.

FORS-enabled devices typically include an integrated twisted multicore optical glass fiber with sensors which may be configured as distributed fiber Bragg grating (FBG) sensors. Light from the light source is used to interrogate the sensors and reflections are analyzed to determine the local deformation of each segment of the optical fiber such that the full three-dimensional shape can be reconstructed. In this way, the full shape of elongated devices can be reconstructed and visualized in real time during the procedure. The FORS enabled devices may be visualized in the context of the patient's anatomy through overlay on images acquired before or during the procedure, like CT scans and X-rays. The amount of images are thus significantly reduced compared to X-ray guided procedures where a new X-ray image is needed every time the elongated device needs to be visualized, leading to a marked reduction in X-ray dose.

The applicant of the present application has developed FORS enabled guidewires and catheters, which have been successfully employed in complex aortic interventional procedures, in particular fenestrated endovascular aortic aneurysm repair (FEVAR) and/or branched endovascular aortic aneurysm repair (BEVAR), as well as in endovascular peripheral lesion repair (EVPLR).

In complex aortic procedures the requirements for mechanical properties of the tip section of the typically used guidewires do not restrict the use of brittle glass fiber sensors which extend up to the distal tip of the elongated device, since navigation often is within large vessels (e.g. aorta), where the guidewire is free to navigate inside the vessel and will typically not encounter obstructions.

In other procedures, such as coronary or various peripheral procedures, such as treatment of peripheral artery disease (PAD), FORS enabled elongated devices are also beneficial for reducing radiation dose. However, in these types of procedures, vessels are much smaller in diameter, and often the trajectory of navigation is very tortuous, i.e. with many bends, and potentially many bifurcations must be accessed to get to the target location, often involving acute angles through which the elongated device must travel. Moreover, these smaller vessels are much more fragile and may be damaged by the tip of the elongated device. Especially in coronary diseases and PAD, angioplasty techniques require crossing a narrowed or occluded section of a blood vessel. For more severe occlusions, crossing a narrowed or occluded section of a blood vessel requires force, and sometimes the tip of the device prolapses, i.e. the device loops and the tip of the device bends back on itself, a phenomenon also called 'knuckling', causing very tight bending in the device tip, i.e. a bending radius < 1 mm. In these cases, the device tip must also be robust enough to withstand this deformation.

For FORS enabled devices, like guidewires, used in peripheral interventional procedures, the tip of the device must be soft and flexible enough to easily navigate through tight bends and acutely angled bifurcations. For example, the tip load of the combination of the inner optical fiber and the outer shell tip of the device must not exceed the limits required for safe navigation, i.e. without damaging the blood vessels, which is around 2-4 gram force for guidewires used in peripheral procedures.

Also during these procedures, the curvature of the optical fiber may become high enough to impede shape sensing or may even become higher than the maximum curvature that the glass fiber can withstand, resulting in failure of the glass material, i.e. breaking or shattering, during use.

In particular, during procedures where a tortuous anatomy must be navigated and/or a narrow lesion or occlusion must be crossed the forces acting on the tip of the device may result in deformations of the device tip which are too extreme for a glass fiber to handle, especially when the device tip is looped or prolapsed. Breaking of the glass fiber will not only impede the use of the FORS technology, but may also result in a patient safety issue if the glass splinters potentially damage the internal structure of the device tip, further altering its mechanical properties.

At the same time, the shape and direction of the tip of the elongated device must still be reconstructed by the FORS technology as accurate as possible even under such challenging circumstances, involving large deformations due to looping and prolapse, resulting in a tight bending radius, such that the need for X-ray guidance can still be avoided.

Conventional movable core guidewires exist, with a movable stainless steel core (for instance Merit Medical's InQwire 0.035" wire), where the core, which is a stiff stainless steel wire, is moveable with respect to the tip coil by manipulating (i.e. pulling back) the proximal end of the wire, such that the core retracts from the tip coil and the distal tip of the guidewire only consists of the flexible coil structure. Furthermore, the tip can be made to prolapse at a well-defined and configurable state coinciding with the distal end of the (retracted) core. Such guidewires are more flexible and atraumatic for the surrounding tissues.

### SUMMARY OF THE INVENTION

It is an object of the present invention to provide an elongated device for medical interventional application, suitable for procedures where a tortuous anatomy must be navigated and/or a narrow lesion or occlusion must be crossed, while at the same time allowing reconstruction of the shape and direction of the tip region of the elongated device by the FORS technology as accurate as possible.

It is a further object to provide a corresponding system comprising a controller and an elongated device.

It is a further object to provide a related computer program executable by a controller.

In a first aspect of the invention, an elongated device for medical interventional application is provided, comprising
a shell comprising a longitudinal lumen, the shell having a main section and a distal terminal section,
an optical fiber arranged in the lumen and having a distal tip, the optical fiber being configured for optical shape sensing,
at least one of the shell and the optical fiber being longitudinally displaceable with respect to the other one of the shell and the optical fiber,
an actuating mechanism configured to displace the at least one of the shell and the optical fiber with respect to the other one of the shell and the optical fiber between an extended state, in which the distal tip of the optical fiber is within the distal terminal section of the shell, and a retracted state, in which the distal tip of the optical fiber is in the main section and out of the distal terminal section of the shell.

The present invention addresses the above-described problem by providing an elongated device for medical interventional application, in which the optical fiber and/or the shell can move relative to the other one so that the distal tip of the optical fiber can be brought out of the distal terminal section of the shell when a sharp bending of the distal terminal section of the device is encountered with the risk of a breaking of the optical fiber. Thus, in the retracted state, the distal terminal section of the shell can prolapse without breaking the optical fiber. Preferably, the distal terminal section of the shell has a bending stiffness lower than that of the main section of the shaft, i.e. the distal terminal section may be softer/more flexible than the main section. In the retracted position, the shape of the elongated device can still be reconstructed up to the location of prolapse by the shape sensing enabled optical fiber, and additionally, after the risk of prolapse of the distal terminal section is again reduced, for instance after crossing an occlusion, the optical fiber can again be advanced into the distal terminal section of the shell so that full shape reconstruction up to the distal terminal section can resume. Preferably, the distal tip of the optical fiber extends up to the outermost distal end of the distal terminal section in the extended state.

The actuating mechanism may be configured to displace only the optical fiber while not moving the shell, or may be configured to displace only the shell without moving the optical fiber, or the actuating mechanism may be configured to displace both, the optical fiber and the shell in order to bring the optical fiber in the extended position or in the retracted position. Thus, the 'retracted state' can be achieved by retracting the optical fiber and/or by advancing the shell.

In particular, the elongated device according to the invention may be a guidewire.

The optical fiber of the elongated device according to the invention may be a multicore optical fiber, wherein the fiber cores may comprise reflective structures responsive to strain in the fiber cores. The reflective structures may be fiber Bragg gratings (FBGs).

It is to be noted that the elongated device according to the invention differs from the known movable core guidewires described above in that the optical fiber of the elongated device according to the invention is not a structural strengthening element, as the stainless steel core wire in the conventional movable core guidewire is, and the optical fiber typically will not have a bending stiffness that is higher than the stiffness of the distal terminal section of the shell of the elongated device according to the invention, whereas the stainless steel core wire in the conventional movable core guidewire is much stiffer than the distal tip of the guidewire.

Preferred embodiments of the invention are defined in the dependent claims and/or are described below.

In an embodiment, the actuating mechanism may be configured to displace the at least one of the shell and the optical fiber with respect to the other one of the shell and the optical fiber continuously between the extended position of the optical fiber and the retracted position of the optical fiber.

In this configuration of the actuating mechanism, it is possible that the distal tip of the optical fiber can assume a continuous spectrum of positions between the extended and the retracted state according to the need of protecting the distal tip section of the optical fiber against breaking.

In another embodiment, the actuating mechanism may be configured to displace the at least one of the shell and the optical fiber with respect to the other one of the shell and the optical fiber incrementally between the extended state and the retracted state.

In this embodiment, the distal tip of the optical fiber may assume a discrete number of spaced apart positions relative to the shell between the extended state and the retracted state. For instance, the actuating mechanism may be configured to displace the shell and/or the optical fiber in steps of 2 mm.

In another embodiment, the actuating mechanism may be configured to allow only the extended state or the retracted state for binary actuation of displacement of the at least one of the shell and the optical fiber with respect to the other one of the shell and the optical fiber.

In this embodiment, the optical fiber has only two stable positions relative to the shell. An advantage of this embodiment is that the optical fiber and/or the shell can be moved between the retracted position and the extended position and vice versa very quickly, while the extended and retracted states or positions are well defined.

In a further embodiment, the movement distance between the extended state and the retracted state is set to a length of the distal terminal section of the shell or slightly more than set length of the distal terminal section.

An advantage here is that the distal tip section including the distal tip of the optical fiber is safely out of the distal terminal section of the shell when the distal terminal section is subject to the risk of prolapsing, e.g. when the distal terminal section of the elongated device crosses an occlusion or a lesion in a blood vessel. On the other hand, the distance between the extended position and the retracted position of the optical fiber is small enough to ensure that shape sensing of the elongated device is enabled as far as possible along the length of the elongated device, i.e. without losing too much shape information along the length of the elongated device.

Preferably, in the retracted state, the distal tip of the optical fiber is in a position along a length of the shell where the distal terminal section of the shell transits to the main section or a position slightly proximally thereof.

In this embodiment, the distal tip of the optical fiber is repositioned in the retracted state at the distal end of the main section of the shell. As the main section has sufficient bending stiffness so that the main section does not prolapse or kink, the optical fiber is well protected against breaking in the retracted state.

In another embodiment, wherein the distal terminal section has a bending stiffness lower than that of the main section, wherein, in the retracted state, the distal tip of the optical fiber is in a position along a length of the shell, in which the bending stiffness of the shell makes a step from a lower bending stiffness to a higher bending stiffness, or in which the first derivative of the bending stiffness as function of state along the length of the shell has a maximum.

In other words, in this embodiment the retracted position of the optical fiber can be collocated with a point along the length of the shell where the bending stiffness of the elongated device increases most abruptly. Due to bending mechanics, this point will be a preferential location for the elongated device to prolapse due to the step difference in bending stiffness. For example, when the distal terminal section of the shell comprises a tip coil, the position where the bending stiffness makes the step from lower to higher bending stiffness may be the position where the tip coil meets the distal end of the main section. In other examples, when the shell of the elongated device comprises a laser cut hypotube, the position along the length of the shell where the bending stiffness makes a step from lower to higher bending stiffness is the position where the design of the laser cuts changes such that the bending stiffness changes. In both examples, the optical fiber in the retracted state will be back far enough to be out of the prolapsing region of the shell and is safe from breaking.

In a further embodiment, the optical fiber is displaceable with respect to the shell, and the actuating mechanism comprises a longitudinally movable fiber actuator engaging the optical fiber, wherein a longitudinal movement of the fiber actuator displaces the optical fiber with respect to the shell.

In this embodiment, the actuating mechanism displaces the optical fiber only by retracting or advancing the optical fiber relative to the shell. In a practical realization, the longitudinally movable fiber actuator may be arranged at or in a docking fin enclosure having a fiber fixation element, where the optical fiber is bonded to the docking fin, such that the optical fiber fixation element can slide linearly back and forth when the slider is actuated. By activating the slider, for example by manually operating the slider or by an automatic control, the optical fiber fixation element moves with respect to the shell of the elongated device, such that the distal tip of the optical fiber is retracted from or advanced towards the distal tip of the distal terminal section of the shell.

In another embodiment, the shell is displaceable with respect to the distal tip of the optical fiber, wherein the actuating mechanism comprises a longitudinally movable shell actuator engaging the shell, wherein a longitudinal movement of the actuator displaces the shell with respect to the optical fiber.

In this embodiment, the shell is configured to be extendable in length such that the distal terminal section of the shell will effectively move forward. This embodiment is advantageous because the optical fiber does not need to move or at least not over a distance as in the previous embodiment where the optical fiber is movable, whereby the buildup of friction and/or potential damage along the optical fiber is reduced.

In a practical embodiment of the previous configuration, the shell may comprise a concentric arrangement of an outer tube and an inner tube, wherein the outer tube comprises the distal terminal section, the optical fiber being arranged in the inner tube, wherein the outer tube is longitudinally displaceable with respect to the inner tube, and wherein the shell actuator engages the outer tube, wherein a longitudinal movement of the shell actuator displaces the outer tube with respect to the optical fiber.

In all embodiments described above, the actuating mechanism may comprise a manually operable control device, or a motorized control device.

The manually operable control device may be a slider arranged at a docking fin enclosure to which the optical fiber is connected in use of the elongated device. The slider may be manipulated by a physician to move the optical fiber back and forth. In other embodiments, the movable fiber actuator or the slider may be motor driven.

In the embodiment above, where the actuating mechanism moves the shell instead of the optical fiber, the shell actuator may comprise a manually operable control device which in the simplest case may be a part of the outer tube of the shell which may be gripped by a physician and pushed forth or pulled back.

In a second aspect of the present invention, a system is provided, comprising an elongated device according to the first aspect and a controller, wherein the controller is configured to automatically activate the actuating mechanism or to provide assistance to a user of the elongated device in operating the actuating mechanism.

When the actuating mechanism comprises a motorized control device, the controller may communicate with the motorized control device and activate same to move the optical fiber and/or the shell into the retracted state or extended state. When the actuating mechanism comprises a manually operable control device, the controller may provide assistance to a user in operating the actuating mechanism. For example, the user can be given a warning, when the tip of the elongated device approaches an occlusion and, thus, a risk of breaking the optical fiber occurs, and/or provided with a hint that the optical fiber and/or the shell are to be moved in order to bring the optical fiber tip out of the distal terminal section of the shell, when a risk of breaking of the optical fiber is encountered.

In an embodiment, the controller may be configured to automatically activate the actuating mechanism or to provide assistance to the user in operating the actuating mechanism in dependence on a curvature of the distal terminal section of the shell.

When the actuating mechanism comprises a motorized control device, it is further preferred according to an embodiment if a controller is provided which is configured to activate the actuating mechanism to displace the shell and/or the optical fiber into the retracted position of the optical fiber in dependence on a curvature of the distal terminal section of the shell.

The curvature of the distal terminal section may be a curvature sensed by the optical fiber, or may be a curvature which is anticipated during navigation of the elongated device, for example, based on the knowledge of some vascular curvatures. That is, the retraction or extension of the optical fiber and/or the shell may be at least partly pre-programmed in a protocol defining the navigation path within the vascular path.

In a further embodiment, the controller may be configured to automatically activate the actuating mechanism or to provide assistance to the user in operating the actuating mechanism when the curvature of the distal terminal section exceeds a first predefined threshold value.

When the actuating mechanism comprises a motorized control device and an increasing curvature or decreasing bending radius of the distal terminal section of the shell is sensed by the optical fiber or anticipated according to a pre-programmed protocol such that a predefined threshold curvature is reached or the risk to be exceeded is encountered, the controller may activate the actuating mechanism to move the optical fiber and/or the shell into the retracted state. Thus, the actuating mechanism may be triggered automatically with the advantage that the maximum curvature at break of the optical fiber will not be reached while the distal tip of the optical fiber is still in the extended state, because it will automatically be brought into the safe retracted state.

In a further embodiment, the controller may be configured to automatically stop the actuating mechanism or to instruct the user to stop operating the actuating mechanism before the fully retracted state is reached, if the curvature of the distal terminal section (18) decreases from the first threshold curvature to a second predefined threshold curvature lower than the first threshold curvature.

In this embodiment, retracting the optical fiber or extending the shell is stopped, if the curvature of the distal terminal section decreases again from the first (upper) threshold curvature by a predefined value or to a lower threshold curvature. That is, the optical fiber is not fully retracted in the retracted state, but only so far as it is necessary to protect the optical fiber against damage. The advantage is that the portion along the distal terminal section of the device which can be shape sensed by the optical fiber is as large as possible without the risk of breaking of the fiber.

In a further embodiment, the controller may be configured to automatically re-activate the actuating mechanism or to instruct the user to resume operating the actuating mechanism, if the curvature increases again from the second threshold curvature to the first threshold curvature.

Thus, the optical fiber and/or the shell are displaced towards the retracted state again, when the curvature is increasing from the lower threshold curvature to the upper threshold curvature. In connection with the previous embodiment, a dynamic retracting and extending mechanism based on curvature feedback between a lower threshold curvature and an upper threshold curvature is thus provided, with the advantage on the one hand that the distal tip of the optical fiber is kept as close as possible to the tip of the distal terminal section so that shape sensing is enabled as close to the distal device tip as possible, and on the other hand the optical fiber is protected against too high curvature.

In a further refinement of the embodiments described before, the first predefined threshold curvature may be set in dependence on a speed by which the elongated device is advanced during use in an interventional application.

The first threshold curvature thus may be dynamic and depend on the advancing speed of the elongated device during use. For example, if the advancing speed is high, i.e. the physician is advancing the elongated device with speed, the threshold curvature may be decreased (or the threshold bending radius increased) such that the actuating mechanism has ample time to be activated. When the elongated device is being advanced slowly, which is often the case close to an occlusion or in tortuous and/or branch vasculature, the threshold curvature may increase (or the threshold bending radius decrease). For instance, when advancing the elongated device at 20 cm/s, the threshold radius may be 4 mm, when the elongated device is advanced at 10 cm/s, the threshold radius may be 3 mm, and when advancing the elongated device at 3 cm/s or lower, the threshold radius may be 2.5 mm.

The advancing speed may be derived from the 3D shape reconstruction itself.

In another embodiment, the automatic actuating mechanism may also interact with a catheter that is shape sensed via a 3D hub, which is a device for visualizing a catheter that is compatible with the elongated device. In this embodiment, an automatic actuating mechanism of the elongated device may be configured in such a way that, when engaged, the optical fiber will always be automatically retracted to the point where the elongated device, e.g. a guidewire, protrudes further than the distal end of the catheter so that the optical fiber is never further distal than the catheter, i.e. where the risk of prolapsing of the distal terminal section of the elongated device is highest.

In a third aspect, a computer program executable by a controller and configured to activate an actuating mechanism of an elongated device of the first aspect, or to provide assistance to a user of said elongated device in operating the actuating mechanism.

### BRIEF DESCRIPTION OF THE DRAWINGS

These and other aspects of the invention will be apparent from and elucidated with reference to the embodiments described hereinafter. In the following drawings:
Fig. 1 shows schematically an elongated device for medical interventional application together with part of components of an optical shape sensing system in a perspective view;
Fig. 2A shows schematically a distal tip region of the elongated device in Fig. 1 in a first state in a longitudinal section view;
Fig. 2B shows schematically the distal tip region of the elongated device in Fig. 1 in a second state in a longitudinal section view;
Fig. 3 shows schematically a detail III in Fig. 1 in a perspective view;
Fig. 4 shows schematically a detail IV in Fig. 1 in a longitudinal section view; and
Fig. 5 shows a diagram of an example of a support profile and the first derivative of the support profile of an elongated device along a length of the elongated device in its distal region.

### DETAILED DESCRIPTION OF THE INVENTION

In the following, embodiments of shape sensing enabled elongated devices for medical interventional application will be described which are especially suitable for navigating tortuous vasculature and for crossing occlusions in a vessel.

In medical interventional applications, such as coronary or other peripheral procedures, such as treatment of peripheral artery disease, Fiber Optic RealShape (FORS) enabled elongated devices, especially FORS enabled guidewires, are beneficial for reducing radiation dose. However, in these types of procedures, vessels are very small in diameter, and often the trajectory of navigation is very tortuous (i.e. with many bends), and potentially many bifurcations must be accessed to get to the target location, often involving acute angles through which the elongated device must travel and, for example, a catheter subsequently must follow. Moreover, these smaller vessels are much more fragile and may be damaged by the tip of the elongated device. Especially in PAD, angioplasty techniques require crossing a narrowed or occluded section in a blood vessel. For more severe occlusions, this typically requires force and sometimes the tip of the wire prolapses, i.e. the wire loops and the tip bends back from itself, a phenomenon also called 'knuckling' which causes very tight bending in the tip of the device, e.g. a bending radius < 1 mm. The tip of the elongated device must also be robust enough to withstand this deformation.

Wang, Jian-Bo et al. "An Effective Guidewire Looping Technique for the Recanalization of Occlusive Segments of Infrapopliteal Vessels", Korean Journal of Radiology 11 (2010), pages 441-448, describe such a transluminal angioplasty technique. As described there, a hydrophilic guidewire is inserted into a proximal branch of the vessel and then a 'U' shape loop is made by rotating and advancing the guidewire continually. The looped guidewire is then advanced into and over occlusive segments of the vessel, whereafter a catheter is crossed over the occlusive segment along the guidewire. The guidewire described there is not equipped with an optical fiber.

When the elongated device, like a guidewire, includes an optical fiber extending up to the distal tip of the elongated device, and when the optical fiber is subject to a loop formation as described above, the curvature of the optical fiber may become too high so that shape sensing is impeded, or, when the curvature of the optical fiber becomes even higher than the maximum curvature that the glass fiber can withstand, this results in failure of the glass material of the optical fiber, i.e. the optical fiber may break or shatter.

The embodiments of the present invention, which will be described in the following, eliminate these drawbacks by providing a new structural design of an elongated device for medical interventional application. The elongated device according to the invention, especially a guidewire, comprises an optical fiber, which may have one or more fiber Bragg gratings, for sensing strain and deformation such that a 3D shape reconstruction of the optical fiber can be performed with an optical interrogator system. The optical fiber especially may be a multicore optical fiber having a plurality of fiber cores, wherein there may be a plurality of outer cores helically spiraled around a center core extending along a longitudinal center axis of the fiber. The optical interrogator system sends laser light through the optical fiber, and then receives and analyzes the returning light that runs through the optical fiber. Twists and bends in the optical fiber influence the wavelength spectrum of the light. By analyzing the wavelength spectra of the returned light, it is possible to reconstruct the 3D shape of the full length of the optical fiber and thus of the elongated device. Thus, radiation-free visualization of the elongated device, for example a guidewire introduced into the vasculature of a patient, in real time and in 3D is enabled.

In order to eliminate the drawbacks of conventional elongated devices, e.g. conventional guidewires comprising an optical fiber, the embodiments to be described below provide that by means of an actuating mechanism, the optical fiber can move back and forth relative to the shell of the elongated device, and/or the shell of the elongated device can move back and forth relative to the optical fiber, such that the distal tip of the optical fiber can be positioned, when necessary, spaced apart from the distal tip of the shell such that the distal tip of the shell can prolapse without the risk of breaking the fiber. The shape of the elongated device can still be reconstructed up to the point of prolapse of the distal terminal section of the shell and additionally, after the risk of prolapse is again reduced (for instance after crossing an occlusion), the optical fiber can again be positioned up to the distal tip of the shell and full shape reconstruction up to the distal tip of the elongated device can resume.

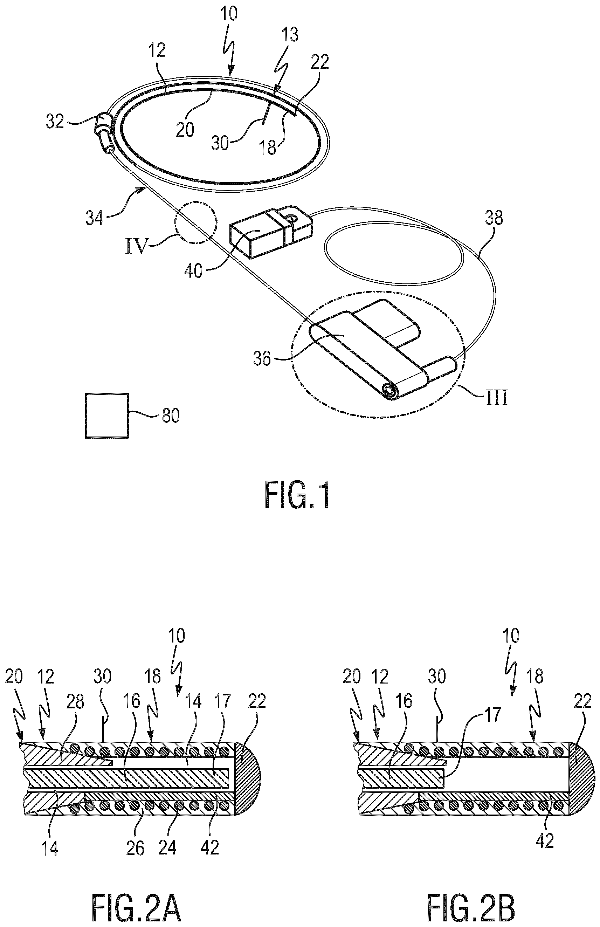

Fig. 1 shows an exemplary embodiment of an elongated device for medical interventional application, which is labeled with general reference numeral 10. The elongated device 10 may be a guidewire.

As shown in more detail in Fig. 2A, the elongated device 10 comprises a shell 12 comprising a lumen 14 extending in longitudinal direction along the length of the device 10. An optical fiber 16, in particular a multicore optical fiber, is arranged in the lumen 14. The optical fiber 16 may be free to slide and rotate in the lumen 14 in longitudinal direction of the shell 12. The optical fiber 16 thus may be a free floating optical fiber in the lumen 14.

The shell 12 comprises a distal terminal section 18 and a main section 20 arranged proximally of the distal terminal section 18. The distal terminal section 18 of the shell terminates at a distal tip 22 forming the distal tip of the device 10. The distal terminal section 18 may comprise a coil 24 embedded in a coating 26, for example a polyurethane coating. The main section 20 of the shell 12 may comprise a tube 28, e.g. a nitinol hypotube, or a tube which may have cuts in its periphery.

Preferably, the distal terminal section 18 of the shell 12 may have a bending stiffness which is lower, preferably much lower than the bending stiffness of the main section 20 of the shell 12. In other words, the distal terminal section 18 is softer/more flexible than the main section 20 such that distal terminal section 18 may prolapse when the elongated device 10 is advanced in a blood vessel and encounters an occlusion or lesion. In Fig. 1, a line 30 symbolizes the transition from the distal terminal section 18 to the main section 20 of the shell 12.

In various embodiments to be described below, the optical fiber 16 may be longitudinally displaceable with respect to the shell, and/or the shell 12 may be longitudinally displaceable with respect to the optical fiber 16.

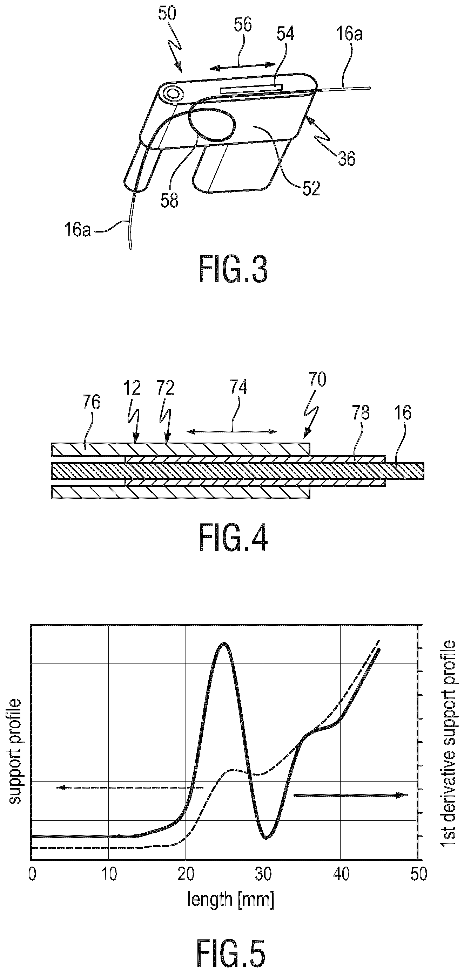

With reference to Fig. 1 again, the elongated device 10 comprises an in-body section 13 illustrated by a dark black line in Fig. 1, which is connected, via an optical connector 32, with a connection wire or cable 34 The connection wire 34 also include an optical fiber (not shown), which may be a proximal portion 16a (Fig. 3) of the optical fiber 16 itself or an optical fiber 16a having the same configuration as the optical fiber 16 and connected with the optical fiber 16 via the optical connector 32. When the optical fiber 16 is displaceable with respect to the shell 12, as provided in some embodiments to be described below, the optical fiber 16a is configured to be displaceable as well, such that a longitudinal displacement of the optical fiber 16a causes the optical fiber 16 in the elongated device 10 to move simultaneously with the fiber 16a. The connection wire 34 connects the optical fiber 16 via fiber 16a to a docking fin 36. The docking fin 36 may be fixed to a side rail of operating table in an operating room (not shown). In the docking fin 36, the optical fiber 16a is fixed relative to the docking fin 36. From the docking fin 36, a further connection wire or cable 38 connects the optical fiber 16 via fiber 16a to a docking base 40. The docking base 40 is connected in optical communication to an optical shape sensing module (not shown) which typically includes an optical interrogator and an analysis system for 3D shape reconstruction.

In the following, embodiments of an actuating mechanism will be described with reference to Figs. 2A and 2B and Figs. 3 and 4. The actuating mechanisms according to these embodiments are configured to displace the optical fiber 16 with respect to the shell 12 and/or to displace the shell 12 with respect to the optical fiber 16 between an extended state, in which the distal tip 17 of the optical fiber 16 is within the distal terminal section 18 of the shell 12, preferably close to the distal tip 22, and a retracted state, in which the distal tip 17 of the optical fiber 16 is in the main section 20 and out of the distal terminal section 18 of the shell 12. The extended state is shown in Fig. 2A, and the retracted state is shown in Fig. 2B. The retracted state can be achieved by retracting the optical fiber 16 relative to the shell 12, and/or by moving the shell 12 forward relative to the optical fiber 16. Insofar, the term "retracted state" does not necessarily mean that the optical fiber 16 has been retracted, but can also mean that the shell 12 has been moved forward.

In the retracted state shown in Fig. 2B, the distal terminal section 18 of the shell 12 can be bent with high curvature or small bending radius, or even prolapse, without the risk that the optical fiber 16 is bent or prolapsed too, and thus a breaking or shattering of the optical fiber 16 is avoided.

Fig. 2A and Fig. 2B further show a ribbon 42 which provides for mechanical stability of the distal terminal section 18, and/or may provide preferential bending direction of the distal terminal section 18, wherein the preferential bending direction is limited to a single bending plane (e.g. the plane of drawing in Fig. 2A/2B).

Fig. 3 shows an exemplary embodiment of an actuating mechanism 50 configured for displacing the optical fiber 16 relative to the shell 12. The actuating mechanism 50 is embodied at or in the docking fin 36. The docking fin 36 comprises a housing 52 into which the optical fiber 16a is introduced and fixed with respect to the docking fin 36 by a fiber fixation element (not shown), i.e. where the optical fiber 16a is bonded to the docking fin 36. The fiber fixation element is an example of a fiber actuator. The actuating mechanism 50 comprises a longitudinally movable slider 54 provided on the housing 52 which is connected to the fiber fixation element. When the slider 54 slides linearly according to a double arrow 56, the fiber fixation element and thus the optical fiber 16a, 16 also slides linearly back and forth. The slider 54 is an example of a control device which may be manually operated by a user in order to move the optical fiber 16 from the extended position according to Fig. 2A into the retracted position according to Fig. 2B.

The actuating mechanism 50 may, in another embodiment, comprise a motorized control device which can be automatically activated as will be described later. In this case, the motorized control device may directly act onto the fiber fixation element within the housing 52 of the docking fin 50, so that the slider 54 is not needed, or the slider 54 may be motorized itself.

Inside the housing 52 of the docking fin 36 behind the optical fiber fixation element, a strain relief section 58 of the optical fiber 16a, for example a loop as shown in Fig. 3, may be provided to accommodate the movement of the optical fiber when the fiber fixation element is moved by operating the slider 54.

Instead of a linearly movable slider 54, the actuating mechanism 50 may comprise a rotatable knob (not shown), the rotation of which is transferred into a linear movement of the fiber fixation element by means of a gear, for example.

The actuating mechanism 50 may be configured to displace the optical fiber 16 with respect to the shell 12 continuously between the extended state and the retracted state, or incrementally between the extended state and the retracted state, or may be configured to allow only the extended state and the retracted state for binary actuation of displacement of the optical fiber 16 with respect to the shell 12. In case of an incremental displacement of the optical fiber 16, the increments may be set in steps of 2 mm, for example. For a manually incrementally operable actuating mechanism 50, the slider 54 may latch at discrete positions along the movement path according to the increments. A bistable mechanism for only binary actuation can be realized in that the slider 54, when actuated, 'springs' from the extended position in the retracted position or vice versa, for example, like a click mechanism of a ballpoint pin.

A movement distance of the optical fiber 16 between the extended state in Fig. 2A and the retracted state in Fig. 2B may be set to a length of the distal terminal section 18 as shown in Fig. 2B, i.e. the distance the slider 54 can linearly move can be set such that the movement distance of the distal tip 17 of the optical fiber 16 corresponds exactly to the length of the distal terminal section 18 which is symbolized by the line 30 in Figs. 2B and 2A, such that the retracting movement of the optical fiber 16 will reposition the distal tip 17 of the optical fiber 16 at the distal end of the tube 28 of the main section 20 of the shell 12. In this position, the optical fiber 16 is relatively safe from high deformations.

Alternatively, the retracted position of the optical fiber 16 can be collocated with a point or position along the length of the shell 12, where the bending stiffness of the shell 12 increases most abruptly. Due to bending mechanics, this point or position will be a preferential location for the shell 12 to prolapse due to a "step" difference in bending stiffness. This can be for instance the position or point, where the coil 24 meets the distal end of the tube 28, or if the tube 28 is a laser cut hypotube, with a change in design of the laser cuts such that the bending stiffness changes. Thus, the retracted optical fiber 16 will be pulled back far enough from the prolapsing region of the shell 12 (distal terminal section 18) and is safe against breaking. Thus, in an embodiment, in particular if the distal terminal section 18 has a bending stiffness lower than that of the main section, the retracted position is the position along the length of the shell 12, in which the bending stiffness of the shell 12 makes a step from a lower bending stiffness to a higher bending stiffness, or in which the first derivative of the bending stiffness as function of position along the length of the shell has a maximum. Thus, the distance, the fiber fixation element in the docking fin 36 can linearly move, can be related to the bending stiffness of the distal terminal section 18 of the shell 12, or the first derivative of the bending stiffness of the distal terminal section 18 such that the movement of the distal tip 17 of the optical fiber 16 into the retracted position coincides with a certain value of the bending stiffness or with a certain change in magnitude of the bending stiffness. This is especially beneficial, because the point or position along the length at which the distal terminal section 18 loops, e.g. the point where the lowest bending radius occurs, often coincides with a sharp change in the bending stiffness.

In case of an elongated device like a FORS enabled guidewire, the transition from the coil 24 to the tube 28 (hypotube) could be such a point. In this embodiment, for example, the distance the slider 54 can be linearly moved could be equal to the (inner) length of the coil 24. For instance, the coil 24 may have a length of 20 mm. There will be a large change in bending stiffness, also referred to as support profile, or the first derivative of the support profile has a maximum, when the coil 24 transitions to the tube 28, as shown in the diagram of Fig. 5. In this case, the distance the slider 54 can linearly move, can be slightly more than 20 mm, for instance 22 mm or 25 mm to ensure the position of the distal tip 17 of the optical fiber 16 is never more distal than the transition point, even if there is some variance in movement due to friction or elasticity in the shell 12 of the device 10.

Another embodiment of an actuating mechanism 70, which is configured to displace the shell 12 relative to the fiber 16 will be described with reference to Fig. 4.

In this embodiment, the shell 12 is displaceable with respect to the distal tip 17 of the optical fiber 16. The actuating mechanism 70 comprises a longitudinally movable shell actuator 72 configured to engage the shell 12. A longitudinal movement of the actuator 72 displaces the shell 12 with respect to the optical fiber 16 as illustrated with a double arrow 74 in Fig. 4.

In this embodiment, the actuating mechanism 70 may be arranged in the connection wire 34 between the in-body section 13 and the docking fin 36 (Fig. 1). The actuating mechanism 70 may comprise a dual concentric tube construction comprising an outer tube 76 and an inner tube 78, wherein the outer tube 76 comprises the distal terminal section 18 including the coil 24. The optical fiber 16 is arranged in the inner tube 78, wherein the shell actuator 72 is configured to engage the outer tube 76, wherein a longitudinal movement of the shell actuator 72 displaces the outer tube 76 with respect to the optical fiber 16. The tubes 76 and 78 can slide over each other and effectively extend the outer tube 76. The shell actuator 72 may, in the simplest configuration, the outer tube 76 itself, wherein a manually operable control device may be the surface of the outer tube 76 that can be gripped by a user.

The actuating mechanism 70 is more easily controllable and closer at hand for the user. Additionally, a free-floating optical fiber 16 inside the lumen 14 of the shell 12, which is restrained only at the base, i.e. in the docking fin 36, will not move along with the distal terminal section 18 of the shell 12, effectively creating the retracted state in Fig. 2B. The advantage of this embodiment is that the length of the optical fiber 16 that moves back and forth inside the shell 12 is not as long as in the embodiment of Fig. 3, reducing the build-up of friction and/or potential damage along the sensor and also maintaining better control of the distal tip 17 of the optical fiber 16, since the optical fiber 16 does not move relative to the (flexible) connection wire 34 part of the shell 12, which is often an elastic material to allow absorbing the torque and stress between the in-body section 13 of the device 10 and the docking fin 36 during manipulation.

Differently from the embodiment of Fig. 3, the distal tip 22 of the device 10 will further extend into a blood vessel when the outer tube 76 is moved in distal direction. Therefore, this embodiment is best employed, when the distal tip 22 of the device 10 has ample room to extend forward and is not yet blocked, for instance by an occlusion.

The actuating mechanism 70 may comprise more than two tubes 76, 78 sliding over each other, such as a more robust housing or an embedded mechanism in a housing that can also be used as a talker, to further improve ease of use.

As in the embodiment of Fig. 3, the actuating mechanism 70 may be manually operable or, in other embodiments may comprise a motorized control device which can be automatically actuated by a controller. Furthermore, the actuating mechanism 70 may be configured to move the outer tube 76 continuously, incrementally between the extended state and the retracted state, or may be bistable to only allow binary actuation from the extended state to the retracted state and vice versa. Further, the movement distance of the outer tube 76 may be set in the same way as described with respect to the actuating mechanism 50 described above.

In the following, an automatic displacement of the optical fiber 16 relative to the shell 12 or displacement of the shell 12 relative to the optical fiber 16 controlled by a controller 80 (Fig. 1) which may be communicatively connected with the actuating mechanism 50 or 70 will be described below.

The controller 80 may be configured to automatically activate the actuating mechanism 50 or 70. Thus, in this embodiment the transition from the extended state in Fig. 2A to the retracted state in Fig. 2B can be triggered automatically. The controller 80 especially may be configured to automatically activate the actuating mechanism 50 or 70 in dependence on a curvature of the distal terminal section 18 of the shell. For instance, if the curvature of the distal tip section 18, which may be sensed by the optical fiber, exceeds a certain threshold curvature, for instance corresponding to a radius below 2.5 mm, the slider 54 or the fiber fixation element in the docking fin 36 will automatically be activated, for instance using a motorized linear actuator, to move the optical fiber 16 and thus the distal tip 17 of the optical fiber 16 into the retracted position as shown in Fig. 2B.

The advantage of such an automatic actuating mechanism is that the bending radius of the optical fiber 16 will not fall below the minimum bending radius at break, while the optical fiber 16 is still at the distal tip 22 of the shell 12, because it will automatically be retracted.

The threshold bending radius (bending radius is the reciprocal of the curvature) can optionally be dynamic and depend on the advancing speed of the elongated device 10 during use, for example, when the elongated device 10 is introduced in the vasculature of a patient. If the elongated device 10 is advanced with high speed, the threshold radius may be increased such that the actuating mechanism 50 has ample time to be activated. When the elongated device 10 is being advanced more slowly, which is often the case close to an occlusion or in tortuous and/or branched vasculature, the threshold radius may decrease accordingly. For instance, when advancing the elongated device 10 at 20 cm/s, the threshold radius may be 4 mm, when advancing the device 10 at 10 cm/s, the threshold radius may be 3 mm, and when advancing the device 10 at 3 cm/s or lower, the threshold radius may be 2.5 mm.

The advancing speed can be derived from the 3D shape reconstruction itself, for instance the advancement of the position of the tip 22 with respect to the launch position, where 3D shape reconstruction starts, or by identifying a fixed shape to use as an anchor point (such as a point, where the device 10 leaves the introducer shell or a relatively sharp bend not far beyond the point where the device 10 enters the vasculature) and derive the movement of the device 10 in time with respect to this fixed point.

The curvature of the distal terminal section 18 of the shell 12 may be sensed by the optical fiber 16, or may be anticipated based on the knowledge of some vascular curvatures, for instance. In this case, the retraction of the optical fiber 16 or extension of the shell 12 may be at least partly pre-programmed in a protocol defining the navigation path within the vascular path.

Additionally, the controller 80 may be programmed to activate the actuating mechanism 50 and/or 70 to retract the optical fiber 16 relative to the shell 12 (or to extend the shell 12 forward relative to the fiber 16) at a certain (upper) threshold curvature as described above, however, not to fully retract the optical fiber 16 to the retracted position (binary actuation). Instead, by monitoring the curvature of the distal terminal section 18 during the retracting process, the retracting is stopped, when the curvature decreases by, or up to, a certain value (the lower threshold curvature), i.e. the optical fiber 16 is not retracted all the way back to the retracted position but only partly, until the curvature is reduced down to the lower threshold curvature, such that the optical fiber 16 is only retracted so far as is needed in that moment.

Furthermore, this mechanism or process can also be programmed to keep the curvature as close as possible to a certain value (or lower of course). In other words, if the curvature increases and the maximum curvature is reached or exceeded (for instance corresponding to 4mm bending radius), then the optical fiber 16 will retract until the curvature is again just below the maximum curvature (corresponding to 4mm bending radius in this example) and stop the retracting temporarily. When the maximum curvature is again reached or exceeded, then the optical fiber 16 is retracted further back, while continuously monitoring the curvature. However, if the curvature reduces again upon further advancing the device 10, because the tortuous section of the vessel has been passed or the vessel is widening and the terminal section 18 is no longer in a state of prolapse, or even if the device 10 is pulled back a bit, then the optical fiber 16 will automatically be moved forward towards the extended state again, until the (upper) curvature threshold is again reached. Thus, this mechanism is providing some sort of curvature feedback based dynamic retracting/extending mechanism to keep the tip 17 of the optical fiber 16 as close to the device distal tip 22 as possible (thereby getting shape information as close to the distal tip 22 as possible) without exceeding the threshold curvature.

In further embodiments, the controller 80 may be configured not to control the motorized control device of the actuating mechanism 50 or 70, but may be configured to provide assistance to a user indicating how the slider 54 in Fig. 3 or the outer tube 76 in Fig. 4 is to be moved, for example according to a pre-programmed protocol based on the navigation path within the vascular path.

Further alternatively, when the elongated device 10 is a guidewire used together with a catheter (not shown) that is shape-sensed for its own via a 3D hub registering the 3D reconstruction of the elongated device with the 3D reconstruction of the catheter, the automatic actuating mechanism 50 or 70 controlled by the controller 80 may also interact with the catheter. In this embodiment, the automatic actuating mechanism 50 or 70 of the elongated device 10 may be configured in such a way that, when the catheter is engaged with the elongated device 10, the optical fiber 16 of the elongated device 10 will always be automatically brought into the retracted state to a point or position, where the elongated device 10 protrudes further than the distal end of the catheter, so that the optical fiber 16 is never further distal than the catheter, i.e. where the risk of prolapsing of the distal terminal section 18 of the device 10 is highest.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Elongated device for medical interventional application, comprising,
a shell (12) comprising a longitudinal lumen (14), the shell (12) having a main section (20) and a distal terminal section (18),
an optical fiber (16) arranged in the lumen (14) and having a distal tip (17), the optical fiber (16) being configured for optical shape sensing,
at least one of the shell (12) and the optical fiber (16) being longitudinally displaceable with respect to the other one of the shell (12) and the optical fiber (16),
an actuating mechanism (50, 70) configured to displace the at least one of the shell (12) and the optical fiber (16) with respect to the other one of the shell (12) and the optical fiber (16) between an extended state, in which the distal tip (17) of the optical fiber (16) is within the distal terminal section (18) of the shell (12), and a retracted state, in which the distal tip (17) of the optical fiber (16) is in the main section (20) and out of the distal terminal section (18) of the shell (12).

2. Elongated device of claim 1, wherein the actuating mechanism (50, 70) is configured to displace the at least one of the shell (12) and the optical fiber (16) with respect to the other one of the shell (12) and the optical fiber (16) continuously or incrementally between the extended state and the retracted state, or the actuating mechanism (50, 70) is configured to allow only the extended state or the retracted state for binary actuation of displacement of the at least one of the shell (12) and the optical fiber (16) with respect to the other one of the shell (12) and the optical fiber (16).

3. Elongated device of claim lor 2, wherein a movement distance between the extended state and the retracted state is set to a length of the distal terminal section (18) of the shell (12) or slightly more than said length of the distal terminal section (18).

4. Elongated device of any one of claims 1 to 3, wherein, in the retracted state, the distal tip (17) of the optical fiber (16) is in a position along a length of the shell (12) where the distal terminal section (18) of the shell transits to the main section (20) or a position slightly proximally thereof.

5. Elongated device of any one of claims 1 to 4, wherein the distal terminal section (18) has a bending stiffness lower than that of the main section (20), wherein, in the retracted state, the distal tip (17) of the optical fiber (16) is in a position along a length of the shell, in which the bending stiffness of the shell (12) makes a step from a lower bending stiffness to a higher bending stiffness, or in which the first derivative of the bending stiffness as function of position along the length of the shell(12) has a maximum.

6. Elongated device of any one of claims 1 to 5, wherein the optical fiber (16) is displaceable with respect to the shell (12), and the actuating mechanism (50) comprises a longitudinally movable fiber actuator configured to engage the optical fiber (16), wherein a longitudinal movement of the fiber actuator displaces the optical fiber (16) with respect to the shell (12).

7. Elongated device of any one of claims 1 to 5, wherein the shell (12) is displaceable with respect to the distal tip (17) of the optical fiber (16), wherein the actuating mechanism (70) comprises a longitudinally movable shell actuator configured to engage the shell (12), wherein a longitudinal movement of the shell actuator displaces the shell (12) with respect to the optical fiber (16).

8. Elongated device of claim 7, wherein the shell (12) comprises a concentric arrangement of an outer tube (76) and an inner tube (78), wherein the outer tube (76) comprises the distal terminal section (18), the optical fiber (16) being arranged in the inner tube (78), wherein the outer tube (76) is longitudinally displaceable with respect to the inner tube (78), and wherein the shell actuator is configured to engage the outer tube (76), wherein a longitudinal movement of the shell actuator displaces the outer tube (76) with respect to the optical fiber (16).

9. Elongated device of any one of claims 1 to 8, wherein the actuating mechanism (50, 70) comprises a manually operable control device.

10. Elongated device of any one of claims 1 to 8, wherein the actuating mechanism (50, 70) comprises a motorized control device.

11. System, comprising an elongated device according to any one of claims 1 to 10 and a controller (80), wherein the controller (80) is configured to activate the actuating mechanism (50, 70) or to provide assistance to a user of the elongated device (10) in operating the actuating mechanism (50, 70), optionally in dependence on a curvature of the distal terminal section (18) of the shell (12), and optionally when the curvature of the distal terminal section (18) exceeds a predefined first threshold curvature.

12. System of claim 11, wherein the controller (80) is configured to stop the actuating mechanism (50, 70) or to instruct the user to stop operating the actuating mechanism (50, 70) before the fully retracted state is reached, if the curvature of the distal terminal section (18) decreases from the first threshold curvature to a second predefined threshold curvature lower than the first threshold curvature.

13. System of claim 12, wherein the controller (80) is configured to re-activate the actuating mechanism (50, 70) or to instruct the user to resume operating the actuating mechanism (50, 70), if the curvature increases from the second threshold curvature to the first threshold curvature.

14. System of any one of claims 11 to 13, wherein the first predefined threshold curvature is set in dependence on a speed by which the elongated device (10) is advanced during use in an interventional application.

15. A computer program executable by a controller and configured to activate an actuating mechanism (50, 70) of an elongated device (10) of any one of claims 1 to 10, or to provide assistance to a user of said elongated device (10) in operating the actuating mechanism (50, 70).
